# EUROPÄISCHE PATENTSCHRIFT

(11) **EP 1 586 586 B1**
(45) Veröffentlichungstag und Bekanntmachung des Hinweises auf die Patenterteilung: **11.03.2009**
(21) Anmeldenummer: 05012488.2
(22) Anmeldetag: 07.12.2001
(51) Int. Cl.: C07K 14/75, A61K 38/36

(54) **Peptide und/oder Proteine sowie ihre Verwendung zur Herstellung eines therapeutischen und/oder präventiven Arzneimittels**
Peptide and/or proteins and their use for the manufacture of a therapeutic and/or preventive medicament
Peptide et/ou protéines ainsi que leur utilisation dans la fabrication d'un médicament thérapeutique et/ou préventif

(30) Priorität: 12.12.2000 AT 20632000
(43) Veröffentlichungstag der Anmeldung: 19.10.2005
(62) Teilanmeldung aus: 01270546.3
(73) Patentinhaber: Fibrex Medical Research & Development GmbH, 1010 Wien (AT)
(72) Erfinder: Petzelbauer, Peter, 1230 Wien (AT)
(74) Vertreter: Schwarz, Albin

(56) Entgegenhaltungen:
- WO-A-93/21962
- DE-A- 19 729 591
- FR-A- 2 795 735
- US-A- 4 927 916
- HARENBERG J ET AL.: "Biodistribution of human fibrinogen-derived peptides in rabits; in: Fibrinogen: Struct. Variants Interact." 1983, DE GRUYTER , BERLIN (DE) , XP008008830 * Seite 271 - Seite 278 *
- HERRICK S ET AL.: "Fibrinogen" THE INTERNATIONAL JOURNAL OF BIOCHEMISTRY & CELL BIOLOGY, Bd. 31, 1999, Seiten 741-746, XP002215500
- SKOGEN W F ET AL: "FIBRINOGEN-DERIVED PEPTIDE BBETA-1-42 IS A MULTIDOMAINED NEUTROPHIL CHEMOATTRACTANT" BLOOD, W.B. SAUNDERS, PHILADELPHIA, VA, US, Bd. 71, Nr. 5, 1988, Seiten 1475-1479, XP008013244 ISSN: 0006-4971
- EVERSE SJ ET AL.: "Conformational Changes in Fragments D and Double-D from Human Fibrin(ogen) upon Binding the Peptide Ligand Gly-His-Arg-Pro-Amide" BIOCHEMISTRY, Bd. 38, 18. Februar 1999 (1999-02-18), Seiten 2941-2946, XP002215499
- MIYAZAKI Y ET AL.: "Fibrinogen derivatives control a novel pathway of transendothelial leukocyte traffic" JOURNAL OF INVESTIGATIVE DERMATOLOGY, Bd. 117, Nr. 2, August 2001 (2001-08), Seite 442, XP008054490
- PETZELBAUER P ET AL.: "The fibrin-derived peptide Bbeta15-42 protects the myocardium against ischemia-reperfusion injury" NATURE MEDICINE, Bd. 11, Nr. 3, März 2005 (2005-03), Seiten 298-304, XP002350673 ISSN: 1078-8956

## Beschreibung

Die Erfindung bezieht sich auf die Verwendung von Peptiden und/oder Proteinen, zur Herstellung eines therapeutischen und/oder präventiven Arzneimittels gegen Entzündungen in der Human- und/oder Veterinärmedizin.

Bisher in Prophylaxe und Therapie verwendete Substanzen zur Hemmung oder Verhinderung von Entzündungsreaktionen, sogenannte Immunsuppresiva, umfassen im wesentlichen zwei verschiedene Gruppen. Erstens Abkömmlinge eines im Körper natürlich vorkommenden Hormons, dem Cortison und zweitens körperfremde Immunsuppressiva, wie Cyclosporin und deren Derivate, Azathioprin, Cyclophosphamid usw. Diesen Substanzen ist eine antiinflammatorische Wirkung gemeinsam, jedoch haben diese Substanzen in der Langzeittherapie erhebliche Nebenwirkungen. Diese Nebenwirkungen limitieren eine langzeitige Therapie, daher werden diese Substanzen alternierend oder in Kombinationen eingesetzt, um Nebenwirkungen auf ein erträgliches Ausmaß zu reduzieren oder um die Therapie überhaupt fortsetzen zu können. Als Nebenwirkung seien die pathologischen Frakturen bei Cortison genannt, die durch den osteoporotischen Effekt des Cortisons verursacht sind oder das Nierenversagen, das durch Cyclosporin hervorgerufen werden kann. Diese Nebenwirkungen sind beiden Verbindungsgruppen obligat, es ist somit lediglich eine Frage der Therapiedauer und Gesamtdosis, wann die Therapie abgesetzt werden muß.

Der vorliegenden Erfindung ist zur Aufgabe gesetzt, neue Pharmazeutika zu schaffen, die geeignet sind, Entzündungsreaktionen zu verhindern oder zu hemmen und lediglich untergeordnete Nebenwirkungen aufweisen. Eine weitere Aufgabe besteht darin, eine Langzeittherapie zur Verfügung zu stellen.

Im folgenden werden die Aminosäuren der erfindungsgemäßen Peptide mit den üblichen Abkürzungen bezeichnet, wobei diese die α-Aminosäuren bezeichnen.

Mit "Analogen" ist ein Peptid gemeint, das sich durch Derivatisierung, Substitution, vorzugsweise homologe Substitution, Deletion und/oder Insertion von der Sequenz des Fibrins und insbesondere den bevorzugten Sequenzen ableitet.

Die erfindungsgemäßen Peptide oder Proteine weisen die allgemeine Formel II 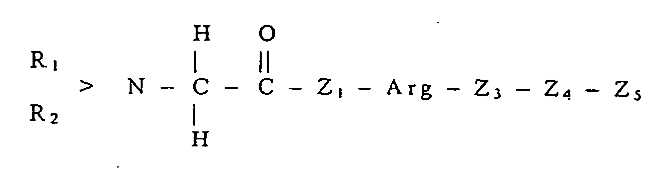 worin R₁ und R₂ gleich oder unterschiedlich, Wasserstoff, einen gesättigten oder ungesättigten Kohlenwasserstoffrest mit 1 bis 3, insbesondere bis 10, Kohlenstoffatomen,
- Z₁: Histidin- oder Prolinrest
- Arg: Argininrest
- Z₃: Prolin- oder Valinrest
- Z₄: Leucin- oder Valinrest
- Z₅: Proteinrest oder Peptidrest, insbesondere mit 2 bis 30 Aminosäuren,
oder Alkoholrest mit 1 bis 3, insbesondere bis 10, Kohlenstoffatomen
oder organischer oder anorganischer Basenrest
bedeuten, auf, sowie deren Salze, als auch z. B. Amide, oder Stoffgemische dieser miteinander und/oder zumindest einem weiteren Stoff zu einer therapeutischen und/oder präventiven Verwendung gegen Entzündungen in der Human- und/ oder Veterinärmedizin, wobei insbesondere nur L-Aminosäuren vorliegen.

Es ist bekannt, daß Fibrinogen die beiden Peptidketten Aalpha und Bbeta aufweist und daß bei entzündlichen Prozessen die Synthese von Fibrinogen stark zunimmt (Herrick et al., The International Journal of Biochemistry & Cell Biology, Bd. 31, 1999, S. 741-746). Ferner wird das Peptid Bβ₁₅₋₄₂ erwähnt (Ikematsu, Rinsho Kensa, Bd. 28, Nr. 1, 1984, S. 20-24). Die Rolle von Fibrinogen-Spaltprodukten bei der Entzündung und Wundheilung wird auch in Blood, Bd. 71, 1988, S. 1475-1479, angesprochen.

Es war vollkommen überraschend, daß die oben definierte Aminosäuresequenz eine antiinflammatorische Wirkung aufweist. Ohne durch diese theoretischen Überlegungen gebunden zu sein, könnte diese Wirkung darauf beruhen, daß das Fibrin über seinen neo-N-Terminus der BbetaKette an Endothelzellen und über die Sequenz der Aalpha-Kette an Zellen im Blutstrom bindet und so zur Adhäsion und Transmigration von Zellen ins Gewebe führt. Diese Verbindungen haben eine Nebenwirkung, und zwar wird die Fibrinbildung gehemmt. Diese Hemmung bedeutet jedoch für den Patienten keinen potentiellen Nachteil, da die Blutgerinnung auch in Abwesenheit von Fibrin bei banalen Verletzungen ausreichend ist. Lediglich bei chirurgischen Eingriffen könnte gegebenenfalls ein Absetzen einer derartigen Therapie zweckmäßig sein. Andere Nebenwirkungen sind im wesentlichen auszuschließen, da diese Substanzen nur mit den natürlichen Liganden interagieren. Weiters wird die natürliche Abwehr durch die Leukozyten im Blut nicht negativ beieinflußt. So bleibt die Zusammensetzung derselben, wie Granulozyten, Lymphozyten und Monozyten unbeeinflußt, so daß der natürliche Abwehrprozeß erhalten bleibt und die Infektabwehr im Blut unverändert bleibt.

Fibrinogen wird in der Leber gebildet und ist in dieser Form biologisch inaktiv und befindet sich normalerweise in Konzentrationen um 3 g/l im Blut. Durch proteolitische Spaltung des Proenzyms Prothrombin wird Thrombin gebildet, welches vom Fibrinogen die Fibrinopeptide A und B abspaltet. Dadurch wird Fibrinogen in seine biologisch aktive Form umgewandelt. Es entstehen Fibrin und Fibrinspaltprodukte.

Thrombin wird bei jeder Aktivierung der Blutgerinnung gebildet, also bei jedem Gewebeschaden, sei dieser entzündlicher, traumatischer oder degenerativer Genese. Die durch Thrombin mediierte Bildung von Fibrin ist prinzipiell ein protektiver Vorgang, um entstandene Defekte im Gefäßsystem rasch abzudichten. Die Bildung von Fibrin ist jedoch auch ein pathogener Vorgang. Die Entstehung eines Fibrinthrombus als auslösende Ursache beim Herzinfarkt ist einer der prominentesten Probleme in der Humanmedizin.

Bisher nicht oder nicht ausreichend untersucht wurde die Rolle von Fibrin bei der Extravasation von Entzündungszellen aus dem Blutstrom ins Gewebe, ein einerseits erwünschter Vorgang bei der Abwehr von pathogenen Mikroorganismen oder Tumorzellen im Gewebe, andererseits aber ein Vorgang, der durch sich selbst Gewebeschaden induziert oder weiter erhält. Fibrin bindet über seinen neo-N-Terminus der Bbeta an Endothelzellen mittels der Sequenz zu Bbeta und an Zellen im Blutstrom mittels der Sequenz Aalpha und führt so zur Adhäsion und Transmigration von Zellen ins Gewebe.

Die erfindungsgemäßen Peptide oder Proteine können die Adhäsion von Zellen aus dem Blutstrom an Endothelzellen der Gefäßwand und/oder ihre nachfolgende Transmigration aus dem Blut ins Gewebe verhindern:

Ein erfindungsgemäßes Peptid oder Protein der allgemeinen Formel II, worin Z₅ ein Peptidrest mit folgender Aminosäuresequenz (SEQ ID NO 1): und
- Z₁: Histidinrest
- Arg: Argininrest
- Z₃: Prolinrest
- Z₄: Leucinrest
bedeuten, verhindert eine Ablagerung bzw. Anheftuhg von Fibrinbruchstücken an die Gefäßwand. Damit können Entzündungszellen an Endothelzellen der Gefäßwandung von Arterien und Venen nicht festgehalten werden und es wird verhindert, daß derartige Zellen an der Gefäßwandung verbleiben und so weiter in das Gewebe gelangen können.

Ein Peptid oder Protein der allgemeinen Formel II, worin Z₅ ein Peptidrest mit folgender Aminisäuresequenz (SEQ ID NO 2): und
- Z₁: Prolinrest
- Arg: Argininrest
- Z₃: Valinrest
- Z₄: Valinrest
bedeuten, bewirkt, daß Zellen des periphären Blutes nicht an Fibrin oder Fibrinbruchstücken anhaften können und damit wird deren Migration im Gewebe verhindert.

Die beschriebenen Spaltprodukte sind auch in der Literatur als Peptid Bbeta und Peptid Aalpha bekannt. Dieser oben angeführte proadhäsive und promigratorische Weg ist ein völlig neuer für das System der Steuerung der Wanderung von Zellen aus dem Blut in das Gewebe. Diese Funktion von Fibrin kann sowohl durch das Peptid Bbetä als auch durch das Peptid Aalpha blockiert werden.

Diese erfindungsgemäßen Peptide bieten sich daher als Therapeutikum bei Mensch und Tier an, um die Wanderung von Zellen aus dem Blut ins Gewebe zu blockieren. Da Fibrin oder andere Produkte des Fibrinogens, die durch proteolytische Spaltung entstehen, wie z. B. Urokinase-Plasminogen-Aktivator gespaltenes Fibrinogen nur spezifisch und regional begrenzt entstehen, also an Stellen von Entzündung, Gerinnungsstörung, Arteriosklerose, Thrombose und/oder Tumorwachstum, handelt es sich hierbei um ein regional beschränkt wirksames Therapeutikum, also pathalogische Nebenwirkungen an anderen Orten sind nicht oder nur in beschränktem Ausmaß zu erwarten.

Bevorzugte und völlig unerwartete Anwendungsbereiche der erfindungsgemäßen Peptide und/oder Proteine liegen in der Herstellung von Arzneimitteln zur Therapie oder Prävention von lokalen und/oder generalisierten Entzündungen des Körpers bei infektiöser Genese, auf Basis einer Autoimmunreaktion, auf Basis einer rheumatischen Erkrankung, auf Basis einer Störung des Immunsystems und/oder auf Basis einer genetischen Erkrankung vor.

Im folgenden wird die Erfindung anhand der Beipiele näher erläutert.

### Beispiel 1:

### Herstellung der Fibrinogenspaltprodukte:

Nicht polymerisierende Degradationsprodukte von Fibrinogen wurden durch Abbau mit Cyanogenbromid gemäß Blombäck et al. (Nature 1968, 218; 130-134) gewonnen. Das so abgebaute Fibrinogen besteht zum Großteil aus einem 63 kD Fragment, und zwar dem N-terminalen Disulfid Knoten, NDSK, und beinhaltet die Aalpha Kette 1-51, Bbeta Kette 1-118 und gamma Kette 1-78. Um NDSK-II zu erhalten (NDSK minus Fibrinopeptide A und B), wurden die N-terminalen Aminosäuren der Aalpha und Bbeta Kette mit Thrombin (20 Einbeiten/1 µg NDSK) in drei Stunden bei Raumtemperatur abgespalten und nachfolgend mit Diisopropylfluorophosphat zur Blockierung der Thrombinaktivität behandelt. Das so erhaltene NDSK-II bestand aus Aalphä Kette 17-51, Bbeta Kette 15-118 und gamma Kette 1-78.

Um NDSK-uPA zu erhalten, wurden 500 µg NDSK eine Stunde bei 37°C mit 200 Einheiten Urokinase-Plasminogen-Aktivator (uPA) der Firma Technoclone, Wien, Österreich, behandelt. Die Reaktion wurde mit 5 mM Phenylmethylsulfonylfluorid abgebrochen. Das so erhaltene NDSK-uPA ist ein NDSK und weist kein Fibrinopeptid B auf.

Für Negativkontrollen wurde eine zweite Fraktion aus den durch Cyanogenbromid Behandlung entstandenen Fibrinogenspaltprodukten, genannt FCB-2 gemäß Nieuwenhuizen et al. (Biochem Biophys Acta 1983, 755; 531 - 533) gewonnen. FCB-2 ist ein 43 kD großes Protein und besteht aus der Aalpha Kette 148-208, der Bbeta Kette 191-305 und der gamma Kette 95-265. Dieses Protein wurde zu Kontrollzwecken ebenso mit Thrombin und mit Diisopropylfluorophosphat versetzt. Es führte jedoch zu keiner Änderung des Proteins (im folgenden FCB-2-thr genannt).

Für weitere Negativkontrollen wurde Kulturmedium (RPMI der Firma Life techn. Inc., Paisky, UK) mit Thrombin, wie oben behandelt und nachfolgend inaktiviert (RPMI-thr) oder mit uPA, wie oben behandelt und inaktiviert (RPMI-uPA).

### Beispiel 2:

Das Peptid Aalpha (SEQ ID NO 2) entspricht den Aminosäuren 1 bis 28 und mit der Sequenz der alpha Kette des Fibrins und ist ident den Aminosäuren 17 bis 45 der Sequenz der Aalpha Kette des Fibrinogens:

Das Peptid Bbeta (SEQ ID NO 1) entspricht den Aminosäuren 1 bis 28 und mit der Sequenz der beta Kette des Fibrins, das ident mit den Aminosäuren 15 bis 43 der Sequenz der Bbeta Kette des Fibrinogens ist, das folgende Sequenz aufweist:

Beide Peptide wurden mittels Festphasen-Peptidsynthese gemäß Merrifield R. B., J. Amer. Chem. Soc. 1963; 85, 2149-2154 mit einem multiplen Peptidsynthesizer unter Anwendung einer Fluorenylmethyloxycarbonyl (FMOC)-Schutzgruppenstrategie gemäß Carpino L. A. und Han. G Y, J. Amer. Chem. Soc. 1981; 37; 3404-3409 synthetisiert. Die Reinigung der Rohpeptide erfolgte mittels präparativer reversed-phase HPLC über eine Nucleosil 100-10, C18 Säule gemäß Engelhart H. und Müller H. Chromatography 1984 19:77 sowie Henschen A., Hupe K. P. und Lottspeich F. High Performance Liquid Chromatography VCH 1985. Als Kontrollpeptide wurden Peptide mit derselben Länge aber mit randomisierter Aminosäuresequenz verwendet.

### Beispiel 3:

### HU-SCID Maus Modell:

Humane Haut wurde auf den Rücken von SCID Mäusen transplantiert und zwei Wochen danach humane Lymphozyten in das Peritoneum injiziert. Es wurde gemäß Petzelbauer et al. (J. Invest. Dermatol. 1996, 107; 576 - 581) verfahren. Es wurde sodann jeweils fünfzehn derartig präparierten Mäusen in die Schwanzvene injiziert:
a) 100 µg humanes NDSK-II
b) 100 µg humanes FCB-2
c) 100 µg Peptid Aalpha
d) 100 µg Peptid Bbeta
e) 100 µg randomisiertes Aalpha
f) 100 µg randomisiertes Bbeta

Vierundzwanzig Stunden danach wurde die humane Haut entnommen und die Anzahl der Entzündungssstellen, ausgedrückt in Zellen pro 0,3 mm², ermittelt und das Mittel mit Standardabweichung bestimmt.

| | |
|---|---|
| Bei a: | 22 +/- 2,8 |
| bei b: | 9 +/- 2,1 |
| bei c: | 4 +/- 1,1 |
| bei d: | 6 +/- 1,1 |
| bei e: | 5 +/- 1,2 |
| bei f: | 7 +/- 1,3 |

Daraus ist abzuleiten, daß NDSK-II zu Entzündungen führt, und es wurde dieses Protein infolge als pathogene Substanz eingesetzt. Die anderen Verbindungen zeigen für sich keine signifikante Erhöhung der Entzündungszellen.

### Vergleichsbeispiel 4:

Fünfzehn Mäusen gemäß Beispiel 3 wurden
100 µg humanes NDSK-II und
100 µg randomisiertes Peptid Aalpha
in die Schwanzvene injiziert. Es wurde gemäß Beispiel 3 weiterverfahren. Es konnten 23 +/- 3,5 Entzündungsstellen pro 0,3 mm² ermittelt werden.

### Vergleichsbeispiel 5:

Fünfzehn Mäusen gemäß Beispiel 3 wurden
100 µg humanes NDSK-II gemäß Beispiel 1 und
100 µg randomisiertes Peptid Bbeta
in die Schwanzvene injiziert. Es wurde gemäß Beispiel 3 weiterverfahren. Es konnten 24 +/- 2 Entzündungsstellen pro 0,3 mm² ermittelt werden.

### Beispiel 6:

Fünfzehn Mäusen gemäß Beispiel 3 wurden
100 µg humanes NDSK-II und
100 µg synthetisiertes Peptid Aalpha
injiziert. Es wurde gemäß Beispiel 3 weiterverfahren. Es konnten 21 +/- 2,2 Entzündungsstellen pro 0,3 mm² ermittelt werden.

### Beispiel 7:

Fünfzehn Mäusen gemäß Beispiel 3 wurden
100 µg humanes NDSK-II und
100 µg synthetisiertes Peptid Bbeta
in die Schwanzvene injiziert. Es wurde gemäß Beispiel 3 weiterverfahren. Es konnten 14 +/- 2 Entzündungsstellen pro 0,3 mm² ermittelt werden.

Den Beispielen 4 bis 7 ist zu entnehmen, daß das Peptid Bbeta die lymphozytäre Entzündung blockiert.

### Vergleichsbeispiel 8:

Humane umbilikale Venen Endothelzellen (HUVEC) wurden mit rotem Floureszenzfarbstoff markiert (Cell Tracker Orange 1 µl/ml, Molecular Probes, Eugene, OR) und auf einer Kollagenmatrix (Collaborative Biomedical Products, Bedford, MA) ausgesät. Nach Konfluenz der Endothelzellen wurden mit grünem Floureszenzfarbstoff (Cell Tracker Green, 1 µl/ml, Molecular Probes der Firma Eugene Origon) markierte periphere Blut mononucleare Zellen (PBMC) (10⁵ Zellen pro 25 mm²) überschichtet. Danach wurden die Zellen für zwölf Stunden auf 37°C inkubiert.

Adhärierende und ins Gel transmigrierte Zellen wurden mit einem Laserscanmikroskop fotografiert, in Pixel transformiert und mittels "NIH image" gemäß Gröger et al. (J. Immunol. Method 1999; 222: 101-109) ausgewertet.

Die Anzahl der adhärenten Zellen pro 0,1 mm² konnte, wie unter Adhäsion angeführt, ermittelt werden. Die Anzahl der migrierten Zellen pro 0,04 mm³ konnte, wie unter Migration angeführt ermittelt werden. Es wurde der Mittelwert von dreimal drei Versuchen gemeinsam mit der Standardabweichung bestimmt.

| | | Adhäsion | Migration |
|---|---|---|---|
| a) RPMI-uPA | 0,1 µg/ml | 40 +/- 4 | 4 +/- 3 |
| | 1,0 µg/ml | 38 +/- 2 | 5 +/- 2 |
| | 10,0 µg/ml | 32 +/- 4 | 5 +/- 1 |
| b) NDSK | 0,1 µg/ml | 31 +/- 18 | 6 +/- 3 |
| | 1,0 µg/ml | 35 +/- 18 | 5 +/- 2 |
| | 10;0 µg/ml | 36 +/- 24 | 6 +/- 3 |
| c) NDSK-II | 0,1 µg/ml | 55 +/- 21 | 12 +/- 5 |
| | 1,0 µg/ml | 67 +/- 31 | 19 +/- 12 |
| | 10,0 µg/ml | 65 +/- 31 | 19 +/- 10 |
| d) NDSK-uPA | 0,1 µg/ml | 58 +/- 3 | 10 +/- 2 |
| | 1,0 µg/ml | 60 +/- 3,5 | 14 +/- 3 |
| | 10,0 µg/ml | 65 +/- 3 | 18 +/- 1,5 |
| e) FCB2 | 0,1 µg/ml | 30 +/-26 | 6 +/- 4 |
| | 1,0 µg/ml | 10 +/- 10 | 3 +/- 2 |
| | 10,0 µg/ml | 21 +/- 7 | 5 +/- 4 |
| f) FCB-2-thr | 0,1 µg/ml | 20 +/-12 | 6 +/- 5 |
| | 1,0 µg/ml | 23 +/- 13 | 7 +/- 5 |
| | 10,0 µg/ml | 26 +/- 11 | 4 +/- 2 |
| g) RPMI-thr | 0,1 µg/ml | 29 +/-15 | 4 +/- 5 |
| | 1,0 µg/ml | 26 +/- 14 | 5 +/- 5 |
| | 10,0 µg/ml | 41 +/- 20 | 5 +/- 4 |

Daraus ist abzuleiten, daß NDSK-II mehr als NDSK-uPA zu signifikanten Migrationen von peripheren Blut-monozellulären Zellen (PBMC) führt und daher pathogen wirkt. Keine der Kontrollen a), b), e), f) und g) führten zu einer signifikanten Migration.

### Beispiel 9:

Die Kollagenmatrix gemäß Beispiel 8 mit der Suspension aus PBMC wurde mit 100 µg NDSK-II und Bbeta oder Bbeta randomisiert versetzt, und es wurde gemäß Beispiel 8 weiterverfahren.

| | | Adhäsion | Migration |
|---|---|---|---|
| a) | kein Zusatz von NDSK-II | 38 +/- 15 | 6 +/- 4 |
| b) | nur 100 µg NDSK-II | 73 +/- 29 | 16 +/- 7 |
| c) | 10 µg Bbeta + NDSK-II | 63 +/-33 | 7 +/- 4 |
| d) | 100 µg Bbeta + NDSK-II | 47 +/- 34 | 5 +/- 4 |
| e) | 1000 µg Bbeta + NDSK-II | 52 +/- 27 | 10 +/- 6 |
| f) | 10 µg Bbeta randomisiert + NDSK-II | 77 +/- 33 | 16 +/- 6 |
| g) | 100 µg Bbeta randomisiert + NDSK-II | 86 +/- 35 | 15 +/- 6 |
| h) | 1000 µg Bbeta randomisiert + NDSK-II | 78 +/- 31 | 13 +/- 8 |

Wie diesen Versuchsergebnissen zu entnehmen, blockiert das Peptid Bbeta Entzündungen.

### Beispiel 10:

Die Kollagenmatrix gemäß Beispiel 8 mit der Suspension aus PBMC wurde mit 100 µg NDSK-II und Aalpha oder Aalpha randomisiert versetzt, und es wurde gemäß Beispiel 8 weiterverfahren.

| | | Adhäsion | Migration |
|---|---|---|---|
| a) | kein Zusatz von NDSK-II | 42 +/- 6 | 10 +/- 1 |
| b) | nur NDSK-II | 96 +/- 11 | 24 +/- 3 |
| c) | 10 µg Aalpha + NDSK-II | 69 +/- 12 | 21 +/- 4 |
| d) | 100 µg Aalpha + NDSK-II | 73 +/- 13 | 15 +/- 6 |
| e) | 1000 µg Aalpha + NDSK-II | 70 +/- 6 | 13 +/- 5 |
| f) | 10 µg Aalpha randomisiert + NDSK-II | 70 +/- 6 | 25 +/- 2 |
| g) | 100 µg Aalpha randomisiert + NDSK-II | 65 +/- 16 | 24 +/- 3 |
| h) | 1000 µg Aalpha randomisiert + NDSK-II | 70 +/- 12 | 26 +/- 3 |

Aus den Versuchsergebnissen läßt sich ableiten, daß das Peptid Aalpha nur teilweise die Migration von PBMC blockiert.

### Beispiel 11:

Da PBMC im wesentlichen aus einem Gemisch von Lymphozyten und Monozyten besteht, wurden im Beispiel 11 reine Lymphozyten anstelle von PBMC (wie in den Beispielen 8 - 10) verwendet.

Die Kollagenmatrix gemäß Beispiel 8 mit Endothelzellen und Lymphzyten wurde mit 100 µg NDSK-uPA bzw. 100 µg NDSK-II und Aalpha bzw. Bbeta versetzt.

| | | Adhäsion | Migration |
|---|---|---|---|
| a) | kein Zusatz | 68 +/- 8 | 16 +/- 3 |
| b) | NDSK-uPA | 143 +/- 11 | 53 +/- 5 |
| c) | NDSK-II | 119 +/- 11 | 43 +/- 4 |
| d) | nur 100 µg Bbeta | 58 +/- 18 | 14 +/- 1 |
| e) | NDSK-uPA + 100 µg Bbeta | 74 +/- 8 | 19 +/- 2 |
| f) | NDSK-II + 1 00 µg Bbeta | 74 +/- 8 | 17 +/- 3 |
| g) | nur 100 µg Aalpha | 77 +/- 4 | 18 +/- 1 |
| h) | NDSK-uPA + 100 µg Aalpha | 131 +/- 4 | 40 +/- 3 |
| i) | NDSK-II + 100 µg Aalpha | 131 +/- 4 | 44 +/- 4 |
| j) | nur 100 µg Bbeta randomisiert | 75 +/- 5 | 19 +/- 1 |
| k) | NDSK-uPA + 100 µg Bbeta | | |
| | randomisiert | 134 +/-13 | 46 +/-4 |
| l) | NDSK-II + 100 µg Bbeta | | |
| | randomisiert | 120 +/- 12 | 42 +/- 4 |

Aus diesen Versuchsergebnissen folgt:
1) daß sowohl NDSK-II als auch NDSK-uPA die Lymphozyten Entzündung fördern,
2) daß das Peptid Bbeta die durch NDSK-II und NDSK-uPA induzierte Lymphozyten Adhäsion und Migration zur Gänze blockiert, hingegen das Peptid Aalpha keinen blockierenden Effekt hat, woraus anzunehmen ist, daß die freie Alpha Kette zur Induktion der Adhäsion und Migration der Lymphozyten nicht benötigt wird.

### Beispiel 12:

Es wurde gemäß Beispiel 11 verfahren, jedoch anstelle der Lymphozyten wurden reine Monozyten verwendet. Es wurde 100 µg NDSK-uPA bzw. 100 µg NDSK-II mit Peptid Aalpha, randomisiertem Aalpha, Bbeta oder randomisierte, Bbeta versetzt.

| | | Adhäsion | Migration |
|---|---|---|---|
| a) | kein Zusatz | 43 +/- 8 | 7 +/- 1 |
| b) | NDSK-uPA | 48 +/- 10 | 10 +/- 2 |
| c) | NDSK-II | 90 +/- 11 | 19 +/- 6 |
| d) | 100 µg Bbeta | 59 +/- 7 | 5 +/- 1 |
| e) | NDSK-uPA + 100 µg Bbeta | 61 +/- 11 | 8 +/- 3 |
| f) | NDSK-II + 100 µg Bbeta | 70 +/- 7 | 7 +/- 5 |
| g) | 100 µg Bbeta randomisiert | 40 +/- 7 | 6 +/- 1 |
| h) | NDSK-uPA + 100 µg Bbeta randomisiert | 45 +/- 5 | 8 +/- 3 |
| g) | NDSK-II + 100 µg Bbeta randomisiert | 92 +/-10 | 20 +/- 7 |
| j) | 100 µg Aalpha | 59 +/- 6 | 5 +/- 1 |
| k) | NDSK-uPA + 100 µg Aalpha | 62 +/- 4 | 8 +/- 5 |
| 1) | NDSK-II + 100 µg Aalpha | 68 +/- 10 | 9 +/- 6 |
| m) | 100 µg Aalpha randomisiert | 58 +/- 7 | 6 +/- 1 |
| n) | NDSK-uPA + 100 µg Aalpha randomisiert | 50 +/- 10 | 10 +/- 4 |
| o) | NDSK-II + 100 µg Aalpha randomisiert | 108 +/- 8 | 21 +/- 5 |

Aus diesen Versuchsergebnissen folgt, daß nur NDSK-II und nicht NDSK-uPA die Wanderung von Monozyten fördert, also sowohl Alpha Kette als auch die Beta Kette ein freies N-terminales Ende aufweisen müssen und die Wanderung der Monozyten blockieren.

### Beispiel 13:

Es wurde gemäß Beispiel 11 verfahren, es wurden reine Lymphozyten verwendet. Es wurde 100 µg NDSK-uPA bzw. 100 µg NDSK-II mit den kurzen Peptidsalzen abgeleitet von Aalpha Gly Pro Arg (Pro)-NH₂ acetat (Aalpha-derivat) oder abgeleitet von Bbeta Gly His Arg Pro-OH acetat (Bbeta-derivat) versetzt.

| | | Adhäsion | Migration |
|---|---|---|---|
| a) | kein Zusatz | 60 +/- 8 | 14 +/- 1 |
| b) | NDSK-uPA | 149 +/- 12 | 57 +/- 5 |
| c) | NDSK-II | 121 +/- 11 | 48 +/- 7 |
| d) | nur 100 µg Bbeta-derivat | 58 +/- 10 | 12 +/- 9 |
| e) | NDSK-uPA + 100 µg Bbeta-derivat | 70 +/- 8 | 16 +/- 3 |
| f) | NDSK-II + 100 µg Bbeta-derivat | 69 +/- 7 | 14 +/- 5 |
| g) | nur 100 µg Aalpha-derivat | 77 +/- 4 | 18 +/- 1 |
| h) | NDSK-uPA + 100 µg Aalpha-derivat | 134 +/- 4 | 48 +/- 5 |
| i) | NDSK-II + 100 µg Aalpha-derivat | 131 +/- 7 | 49 +/- 6 |
| j) | nur 100 µg Bbeta-derivat | | |
| | randomisiert | 70 +/- 5 | 14 +/- 7 |
| k) | NDSK-uPA + 100 µg Bbeta-derivat | | |
| | randomisiert | 130 +/- 12 | 49 +/- 6 |
| 1) | NDSK-II + 100 µg Bbeta-derivat randomisiert | 120 +/- 10 | 55 +/- 8 |

Aus diesem Experiment ergibt sich, daß bei Hemmung der Lymphozytenmigration diese kurzen Peptide in entsprechender kontinuierlicher Zugabe gleich wirksam wie die langen Peptide sind.

### Beispiel 14:

Es wurde gemäß Beispiel 12 verfahren, es wurden reine Monozyten verwendet. Es wurde 100 Mg NDSK-uPA bzw. 100 µg NDSK-II mit den kurzen Peptidsalzen Aalpha Gly Pro Arg (Pro)-NH₂ acetat (Aalpha-derivat) oder Bbeta Gly His Arg Pro-OH acetat (Bbeta-derivat) versetzt.

| | | | |
|---|---|---|---|
| | | Adhäsion | Migration |
| a) | kein Zusatz | 40 +/- 8 | 5 +/- 1 |
| b) | NDSK-uPA | 54 +/- 9 | 7 +/- 2 |
| c) | NDSK-II | 85 +/- 11 | 22 +/- 6 |
| d) | 100 µg Bbeta-derivat | 52 +/- 7 | 6 +/- 1 |
| e) | NDSK-uPA + 1 00 µg Bbeta-derivat | 61 +/- 11 | 8 +/- 3 |
| f) | NDSK-II + 100 µg Bbeta-derivat | 68 +/- 7 | 8 +/- 4 |
| g) | 100 µg Bbeta-derivat randomisiert | 40 +/- 7 | 6 +/- 1 |
| h) | NDSK-uPA + 100 µg Bbeta-derivat randomisiert | 44 +/- 6 | 8 +/- 2 |
| i) | NDSK-II + 100 µg Bbeta-derivat randomisiert | 92 +/- 10 | 23 +/- 7 |
| j) | 100 µg Aalpha-derivat | 50 +/- 5 | 4 +/- 4 |
| k) | NDSK-uPA + 100 µg Aalpha-derivat | 60 +/- 5 | 7 +/- 6 |
| l) | NDSK-II + 100 µg Alpha-derivat | 64 +/- 11 | 8 +/- 2 |
| m) | 100 µg Aalpha-derivat randomisiert | 54 +/- 10 | 6 +/- 3 |
| n) | NDSK-uPA + 100 µg Aalpha-derivat randomisiert | 50 +/- 10 | 10 +/- 4 |
| o) | NDSK-II + 100 µg Aalpha-derivat randomisiert | 99 +/- 8 | 21 +/- 7 |

Aus diesem Experiment ergibt sich, daß bei Hemmung der Monozytenmigration diese kurzen Peptide in entsprechender kontinuierlicher Zugabe gleich wirksam wie die langen Peptide sind.

## Patentansprüche

1. Verwendung von Peptiden oder Proteinen der allgemeinen Formel II 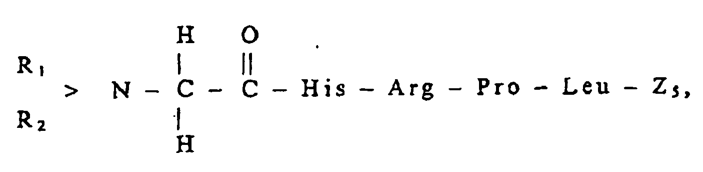 worin R₁ und R₂ gleich oder unterschiedlich, Wasserstoff, einen gesättigten oder ungesättigten Kohlenwasserstoffrest mit 1 bis 10, insbesondere bis 3, Kohlenstoffatomen,
Z₅ ein Proteinrest oder Peptidrest, insbesondere mit 2 bis 30 Aminosäuren, oder Alkoholrest mit 1 bis 10, insbesondere bis 3, Kohlenstoffatomen
oder organischer oder anorganischer Basenrest
bedeuten sowie deren Salze, als auch z. B. Amide, oder Stoffgemische dieser miteinander und/oder zumindest einem weiteren Stoff zur Herstellung eines Arzneimittels zur therapeutischen und/oder präventive Verwendung gegen Entzündungen in der Human- und/oder Veterinärmedizin.

2. Verwendung von Peptiden oder Proteinen der allgemeinen Formel II nach Anspruch 1, worin Z₅ ein Peptidrest mit folgender Aminosäuresequenz (SEQ ID NO 1): bedeutet zur Herstellung eines Arzneimittels zur therapeutischen und/oder präventiven Verwendung gegen Entzündungen in der Human- und/oder Veterinärmedizin.

3. Verwendung von Peptiden und/oder Proteinen nach Anspruch 1 oder 2 zur Herstellung eines Arzneimittels zur Therapie von lokalen und/oder generalisierten Entzündungen des Körpers infektiöser Genese.

4. Verwendung von Peptiden und/oder Proteinen nach Anspruch 1, 2 oder 3 zur Herstellung eines Arzneimittels zur Therapie von lokalen und/oder generalisierten Entzündungen des Körpers auf Basis einer Autoimmunreaktion.

5. Verwendung von Peptiden und/oder Proteinen nach einem der Ansprüche 1 bis 4 zur Herstellung eines Arzneimittels zur Therapie von lokalen und/oder generalisierten Entzündungen des Körpers auf Basis einer rheumatischen Erkrankung.

6. Verwendung von Peptiden und/oder Proteinen nach einem der Ansprüche 1 bis 5 zur Herstellung eines Arzneimittels zur Therapie von lokalen und/oder generalisierten Entzündungen des Körpers auf Basis einer Störung des Immunsystems.

7. Verwendung von Peptiden und/oder Proteinen nach einem der Ansprüche 1 bis 6 zur Herstellung eines Arzneimittels zur Therapie von lokalen und/oder generalisierten Entzündungen des Körpers auf Basis einer genetischen Erkrankung.

## Claims

1. The use of peptides or proteins of the general formula II 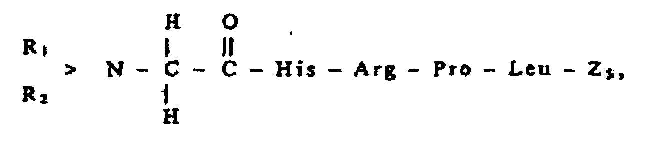 wherein R₁ and R₂, being equal or different, denote hydrogen, a saturated or unsaturated hydrocarbon residue comprising from 1 to 10, in particular up to 3, carbon atoms,
Z₅ denotes a protein residue or a peptide residue, in particular comprising from 2 to 30 amino acids,
or an alcohol residue comprising from 1 to 10, in particular up to 3, carbon atoms,
or an organic or inorganic base residue,
as well as the salts thereof, and, f.i., also amides, or mixtures with each other and/or with at least one further substance for the preparation of a pharmaceutical composition for therapeutic and/or preventive use against inflammations in human and/or veterinary medicine.

2. The use of peptides or proteins of the general formula II according to claim 1,
wherein Z₅ denotes a peptide residue comprising the following amino acid sequence (SEQ ID NO 1): for the preparation of a pharmaceutical composition for therapeutic and/or preventive use against inflammations in human and/or veterinary medicine.

3. The use of peptides and/or proteins according to claim 1 or 2 for the preparation of a pharmaceutical composition for the therapy of local and/or generalized inflammations in the body that are of infectious genesis.

4. The use of peptides and/or proteins according to claim 1, 2 or 3 for the preparation of a pharmaceutical composition for the therapy of local and/or generalized inflammations in the body, based upon an auto-immune reaction.

5. The use of peptides and/or proteins according to any of claims 1 to 4 for the preparation of a pharmaceutical composition for the therapy of local and/or generalized inflammations in the body, based upon a rheumatic disease.

6. The use of peptides and/or proteins according to any of claims 1 to 5 for the preparation of a pharmaceutical composition for the therapy of local and/or generalized inflammations in the body, based upon a disorder in the immune system.

7. The use of peptides and/or proteins according to any of claims 1 to 6 for the preparation of a pharmaceutical composition for the therapy of local and/or generalized inflammations in the body, based upon a genetic disease.

## Revendications

1. Utilisation de peptides ou de protéines de formule générale II 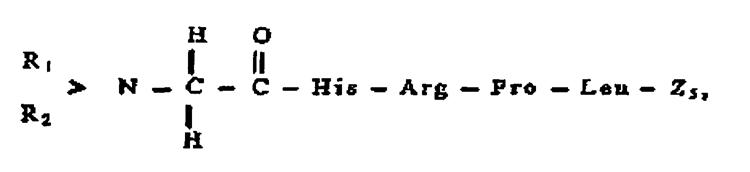 dans laquelle R₁ et R₂, identiques ou différents, représentent un atome d'hydrogène, un radical hydrocarboné saturé ou insaturé ayant de 1 à 10, en particulier jusqu'à 3 atomes de carbone,
Z₅ représente un radical protéinique ou radical peptidique, ayant en particulier de 2 à 30 acides aminés,
ou un radical alcool ayant de 1 à 10, en particulier jusqu'à 3 atomes de carbone
ou un radical de base organique ou inorganique
ainsi que de leurs sels comme également par exemple les amides, ou de mélanges de substances de ces derniers entre eux, et/ou d'au moins une substance supplémentaire pour préparer un médicament destiné à une utilisation thérapeutique et/ou préventive contre les inflammations dans la médecine humaine et/ou vétérinaire.

2. Utilisation de peptides ou protéines de formule générale II selon la revendication 1, dans laquelle Z₅ représente un radical peptidique ayant la séquence d'acides aminés suivante (SEQ ID NO:1) : pour la préparation d'un médicament destiné à une utilisation thérapeutique et/ou préventive contre les inflammations dans la médecine humaine et/ou vétérinaire.

3. Utilisation de peptides et/ou de protéines selon la revendication 1 ou 2 pour préparer un médicament destiné à la thérapie d'inflammations locales et/ou généralisées du corps, d'origine infectieuse.

4. Utilisation de peptides et/ou de protéines selon la revendication 1, 2 ou 3 pour préparer un médicament destiné à la thérapie d'inflammations locales et/ou généralisées du corps, sur la base d'une réaction auto-immune.

5. Utilisation de peptides et/ou de protéines selon l'une quelconque des revendications 1 à 4 pour préparer un médicament destiné à la thérapie d'inflammations locales et/ou généralisées du corps, sur la base d'une maladie rhumatismale.

6. Utilisation de peptides et/ou de protéines selon l'une quelconque des revendications 1 à 5 pour préparer un médicament destiné à la thérapie d'inflammations locales et/ou généralisées du corps, sur la base d'un trouble du système immunitaire.

7. Utilisation de peptides et/ou de protéines selon l'une quelconque des revendications 1 à 6 pour préparer un médicament destiné à la thérapie d'inflammations locales et/ou généralisées du corps, sur la base d'une maladie génétique.


### SEQUENCE LISTING

<110> FIBREX Medical Research & Development GmbH Petzelbauer, Peter
<120> Peptide und/oder Proteine sowie verwendung desselben zur Herstellung eines therapeutischen und/oder präventiven Arzneimittels
<130> 2760 PCT
   <140> PCT/AT 01/00387
   <141> 2001-12-07
   <150> AT A 2063/2000
   <151> 2000-12-12
   <160> 2
   <170> PatentIn version 3.1
<210> 1
   <211> 28
   <212> PRT
<213> Artificial sequence;
<220>
   <221> source
   <223> /note="entspricht den Aminosäuren 1-28 mit der sequenz der Bbeta Kette des Fibrins"
   <400> 1
   Gly His Arg Pro Leu Asp Lys Lys Arg Glu Glu Ala Pro Ser Leu Arg
   1               5                   10                  15
   Pro Ala Pro Pro Pro Ile Ser Gly Gly Gly Tyr Arg
               20                  25
<210> 2
   <211> 28
   <212> PRT
   <213> Artificial Sequence
<220>
   <221> source
   <223> /note="entspricht den Aminosäuren 1-28 mit der sequenz der Aalpha
   Kette des Fibrins"
<400> 2
   Gly Pro Arg Val Val Glu Arg His Gln Ser Ala Cys Lys Asp Ser Asp
   1               5                   10                  15
   Trp Pro Phe Cys Ser Asp Glu Asp Trp Asn Tyr Lys
               20                  25